(19) Europäisches Patentamt
European Patent Office
Office européen des brevets

(11) EP 2 459 155 B1

(12) FASCICULE DE BREVET EUROPEEN

(45) Date de publication et mention de la délivrance du brevet:
**14.01.2015 Bulletin 2015/03**

(21) Numéro de dépôt: **10752082.7**

(22) Date de dépôt: **22.07.2010**

(51) Int Cl.:
*A61Q 17/04* (2006.01)    *A61Q 19/00* (2006.01)
*A61K 8/37* (2006.01)    *A61K 8/34* (2006.01)
*A61K 8/73* (2006.01)    *A61K 8/60* (2006.01)
*A61K 8/92* (2006.01)    *A61K 8/06* (2006.01)
*A61Q 19/04* (2006.01)    *A61Q 5/02* (2006.01)
*A61Q 5/00* (2006.01)

(86) Numéro de dépôt international:
**PCT/FR2010/051556**

(87) Numéro de publication internationale:
**WO 2011/015759 (10.02.2011 Gazette 2011/06)**

(54) **EMULSION HUILE-DANS-EAU À PROPRIÉTÉS SENSORIELLES AMÉLIORÉES**

ÖL-IN-WASSER-EMULSION MIT VERBESSERTEN SENSORISCHEN EIGENSCHAFTEN

OIL-IN-WATER EMULSION HAVING IMPROVED SENSORY PROPERTIES

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **27.07.2009 FR 0955247**

(43) Date de publication de la demande:
**06.06.2012 Bulletin 2012/23**

(73) Titulaire: **Société d'Exploitation de Produits pour les Industries Chimiques SEPPIC**
**75007 Paris (FR)**

(72) Inventeurs:
• **AMALRIC, Chantal**
  **F-81700 Blan (FR)**
• **SHEN, Juanshu**
  **F-81100 Castres (FR)**

• **GUILBOT, Jérôme**
  **F-81100 Castres (FR)**
• **ROLLAND, Hervé**
  **F-81100 Castres (FR)**
• **GORCE, Agnès**
  **F-13009 Marseille (FR)**
• **KERVERDO, Sébastien**
  **F-94300 Vincennes (FR)**

(74) Mandataire: **Conan, Philippe Claude**
**L'Air Liquide SA**
**Direction de la Propriété Intellectuelle**
**75, quai d'Orsay**
**75321 Paris Cedex 07 (FR)**

(56) Documents cités:
**WO-A1-01/58578      WO-A1-95/13863**
**DE-C1- 19 730 423    FR-A1- 2 803 537**
**US-B1- 6 348 202**

EP 2 459 155 B1

**Description**

La présente invention a pour objet de nouvelles compositions se présentant sous la forme d'émulsions huile-dans-eau, ainsi que leurs procédés de préparation.

L'invention trouve application préférentiellement dans le domaine cosmétique et dermocosmétique, dans le domaine dermopharmaceutique et pharmaceutique, notamment dans l'utilisation pour le soin, la protection et le nettoyage de la peau, des cheveux et du cuir chevelu, mais également dans le domaine de l'industrie textile, par exemple pour le traitement de fibres textiles synthétiques ou naturelles tissées ou tricotées ou encore dans le domaine de l'industrie papetière par exemple pour la fabrication de papier à usage sanitaire ou domestique.

Dans la mise au point de formulations destinées à traiter et/ou à protéger la peau et/ou les muqueuses et/ou le cuir chevelu, et de façon générale des produits de soin corporel, les propriétés sensorielles constituent un critère important pour leur succès commercial. En effet, le choix du consommateur final se fonde aussi bien sur des critères de performances techniques que sur des critères sensoriels et de bien-être immédiat que lui procurent les formulations cosmétiques appliquées sur la peau. De plus, l'utilisation d'ingrédients d'origine naturelle pour la préparation des formulations cosmétiques est également un élément du succès commercial de ces produits car elle permet l'obtention de labels délivrés par des organismes professionnels, qui certifient leur origine naturelle. Dans la préparation de ces formulations cosmétiques, on peut utiliser des corps gras d'origine végétale comme phases grasses, des agents épaississants et/ou gélifiants et/ou stabilisants d'origine naturelle, des tensioactifs préparés à partir de matières premières d'origine végétale comme agents émulsionnants. Parmi les tensioactifs utilisés comme agents émulsionnants et préparés à partir de matières premières d'origine végétale, les formulateurs utilisent couramment les esters de glycérol et d'acides gras d'origine végétale, comme le stéarate de glycérol, les savons comme le stéarate de sodium, les tensioactifs dérivés de sucres et d'acides gras d'origine végétale, comme les esters gras de sucrose, ainsi que les tensioactifs dérivés de sucres et d'alcools gras d'origine végétale qui ont été développés depuis plus d'une vingtaine d'années et dont le succès commercial n'est plus à démontrer. De tels agents émulsionnants sont constitués d'un mélange d'alkyl polyglycosides et d'alcools gras et se présentent sur le marché soit sous forme solide, qu'il s'agisse d'écailles ou de perles, soit sous forme liquide à température ambiante selon les alcools gras mis en oeuvre. Ils sont par exemple décrits dans les demandes internationales publiées sous les numéros WO 92/06778, WO 95/13863, WO 96/37285 ou WO 00/56438 ou encore dans la demande de brevet français publiée sous le numéro FR 2 830 464.

La demande de brevet de brevet français publiée sous le numéro FR 2 803 537 divulgue plus particulièrement des compositions comprenant de 5 à 95 parties en poids d'un mélange d'alkylpolyglycosides constitué des produits de réaction d'un saccharide et du 1,12 octadécanediol, et de 95 à 5 parties en poids de 1,12-octadécanediol. Elles permettent de préparer des émulsions opaques et stables, appropriées à la préparation de formulations cosmétiques moussantes. Cette publication divulgue aussi une composition cosmétique utilisée comme un lait démaquillant moussant comprenant, pour 100% de sa masse, 4% massique d'un mélange de 1,12-octadécanediol et de d'alkylpolyglycosides dont la chaîne alkyle est constituée par le radical 12-hydroxyoctadécyloxy, 4% massique d'huile de jojoba, 3% massique d'un agent épaississant à base de copolymères acryliques commercialisé sous l'appellation CAPIGEL™98, 7% massique de lauryl éther sulfate de sodium et le reste à 100% massique d'eau. L'huile de jojoba est un mélange d'esters ; chaque molécule se composant d'un acide gras linéaire et d'un alcool gras linéaire, chacun comprenant de 18 à 22 atomes de carbone, liés entre eux par une liaison ester *(*D.J. Understander, E.A. Oelke, A.R. Kaminski, J.D. Doll, S.M. Combs, and C.V. Hanson, « Jojoba » in Alternative Field Crops Manual, 1990*)*. Les compositions émulsionnantes décrites dans FR 2 803 537 sont appropriées aux compositions cosmétiques moussantes, en permettant d'améliorer la stabilité et l'opacité, propriété visuelle, desdites compositions.

La publication internationale WO 95/13863 divulgue un concentré comprenant un mélange émulsionnant d'alkylpolyglycosides et d'alcools gras destiné à préparer des émulsions à stabilité améliorée et à procurer à ladite émulsion un effet nacré, à savoir un effet visuel consistant à conférer à la composition cosmétique un effet irisé, moiré, métallisé ou opacifiant. La stabilité que confère un tel concentré émulsionnant et nacrant peut permettre au formulateur d'éviter d'utiliser des agents stabilisants comme des polymères épaississants usuellement employés pour parfaire à la stabilité des émulsions.

La publication US 6,348, 202 B1 divulgue la préparation de compositions cosmétiques auto-bronzantes comprenant de la dihydroxyacétone et des émulsionnants non ioniques, lesdits émulsionnants non ioniques étant constitués par un mélange d'alkyl et/ou d'alkenyl oligoglycosides et de polyols poly-12-hydroxystéarates. L'utilisation de tels émulsionnants non ioniques permet de conférer à la composition cosmétique auto-bronzante une stabilité améliorée et d'éviter la décomposition chimique de la dihydroxyacétone pendant un stockage à haute température de la composition cosmétique auto-bronzante.

Cependant, l'application sur la peau de formulations cosmétiques, comprenant des corps gras d'origine végétale et/ou des agents épaississants et/ou gélifiants et/ou stabilisants d'origine naturelle, et/ou des agents émulsionnants dérivés de sucres et d'alcools gras d'origine végétale, et/ou des agents émulsionnants dérivés de sucres et d'acide gras d'origine végétale, et/ou des agents émulsionnants à base d'esters d'acides gras d'origine végétale et de glycérol, ne donne pas des résultats sensoriels satisfaisants.

Les propriétés sensorielles d'une formulation cosmétique pour le soin corporel se caractérisent par plusieurs critères, parmi lesquels on peut citer la sensation de douceur procurée à l'utilisateur lors de l'application de la composition sur la peau, la consistance de la composition et le phénomène dit de "savonnage". La consistance d'une formulation cosmétique se caractérise par sa résistance à l'écoulement. Elle est déterminée de façon empirique, par observation visuelle de l'écoulement de la formulation cosmétique sous l'effet de la gravité et/ou par la mesure d'une force maximale de compression mise en oeuvre à l'aide d'un texturomètre muni d'une sonde de mesure et relié à un logiciel de traitement des données. Le phénomène dit de "savonnage" mentionné plus haut, se caractérise par l'apparition d'un film blanchâtre sur la peau, lors de l'application de la formulation sur la peau. Ce critère est évalué par observation visuelle de son existence et/ou de la vitesse de son apparition. Il peut être réduit par l'ajout d'additifs dans des formulations cosmétiques, comme des huiles de silicones tel que décrit par E.W. Flick dans l'ouvrage de référence « Cosmetic additives - an industrial guide », William Andrew Publishing/Noyes, 1991.

Il est également divulgué dans la publication internationale WO 94/01073, que la préparation de formulations destinées à traiter et/ou à protéger la peau se présentant sous la forme d'émulsions triples et comprenant des polymères de silicone comportant une chaîne polyoxyéthylénée et/ou polyoxypropylénée, permettait d'éviter ce phénomène de "savonnage" décrit précédemment.

La demande de brevet européen publiée sous le numéro EP 0 958 856 A1 décrit également des formulations se présentant sous la forme d'émulsions triples eau-dans-huile-dans-eau, et se caractérisant par la présence d'au moins un élastomère organopolysiloxane partiellement ou totalement réticulé comportant une chaîne polyoxyéthylénée et/ou polyoxypropylénée, qui ne présentent pas le phénomène de "savonnage" lors de leur application sur la peau.

Ces solutions techniques présentent cependant comme inconvénient d'introduire des ingrédients constitutifs des formulations cosmétiques qui ne sont pas d'origine naturelle.

Les inventeurs ont donc cherché à développer de nouvelles compositions comprenant des ingrédients d'origine naturelle et présentant des propriétés sensorielles améliorées c'est-à-dire des compositions :

- qui restent homogènes au stockage à température ambiante après une durée minimale de un mois,
- qui sont caractérisées par un indice de douceur, dont le protocole est décrit dans le paragraphe II-3 de la partie expérimentale du présent exposé, supérieur à une valeur de 7,0,
- qui sont évaluées comme satisfaisant au critère de consistance, avant son application sur la peau par un panel dûment entrainé de 11 personnes selon les conditions opératoires du test d'analyse sensorielle dont le protocole est décrit dans le paragraphe II-2 de la partie expérimentale du présent exposé,
- qui sont évaluées comme « non savonnantes » pendant la phase d'application sur la peau par un panel dûment entrainé de 11 personnes selon les consitions opératoires du test d'analyse sensorielle dont le protocole est décrit dans le paragraphe II-1 de la partie expérimentale du présent exposé.

C'est pourquoi, selon un premier aspect, l'invention a pour objet une composition se présentant sous la forme d'une émulsion huile-dans-eau comprenant pour 100% de sa masse :

a) - De 0,1% à 10% massique d'un système émulsionnant (A) consistant pour 100% de sa masse en :

(i) - De 5% massique à 95% massique du mélange des produits de la réaction d'un sucre réducteur et du 1,12-octadécanediol constitué essentiellement d'hydroxyoctadécyl polyglycosides de formule (I) :

$$HO\text{-}R\text{-}O\text{-}(G)_n\text{-}H \qquad (I)$$

et de polyglycosyloctadécylpolyglycosides de formule (II) :

$$H\text{-}(G)_m\text{-}O\text{-}R\text{-}O\text{-}(G)_p\text{-}H \qquad (II),$$

formules (I) et (II) dans lequelles G représente le reste dudit sucre réducteur et R représente le radical divalent octadécane-diyl-1,12 et dans lesquelles n, m et p, identiques ou différents, représentent chacun, indépendamment les uns des autres, un nombre décimal compris entre 1 et 5 ;
(ii) - De 95% massique à 5% massique de 1,12-octadécanediol ;

b) - De 0,01% à 5% massique d'au moins un agent épaississant et/ou gélifiant choisi parmi les agents épaississants et/ou gélifiants d'origine naturelle ;
c) - De 10% à 50% massique d'une phase grasse (P2) constituée d'une ou plusieurs huiles végétales à base de monoglycérides et/ou de diglycérides et/ou de triglycérides ; et

d) - De 89,89% massique à 35% massique d'une phase aqueuse (P1) cosmétiquement acceptable.

Dans la définition de formule (I) telle que définie précédemment, n est un nombre décimal qui représente le degré moyen de polymérisation du reste G. Lorsque n est un nombre entier, $(G)_n$ est le reste polymérique de rang n du reste G. Lorsque n est un nombre décimal, la formule (I) représente un mélange de composés :

$$a_1 \, HO\text{-}R\text{-}O\text{-}G\text{-}H + a_2 \, HO\text{-}R\text{-}O\text{-}(G)_2\text{-}H + a_3 \, HO\text{-}R\text{-}O\text{-}(G)_3\text{-}H + ... + a_q \, HO\text{-}R\text{-}O\text{-}(G)_q\text{-}H$$

avec q représentant un nombre entier compris entre 1 et 5 et dans les proportions molaires $a_1$, $a_2$, $a_3$,... aq telles que :

$$\sum_{q=5}^{q=1} a_q = 1 \; ; a_1 > 0$$

Dans la définition de la formule (II) telle que définie précédemment m et p sont des nombres décimaux qui représentent le degré moyen de polymérisation du reste G. Lorsque m est un nombre entier, $(G)_m$ représente le reste polymérique de rang m du reste G. Lorsque p est un nombre entier, $(G)_p$ représente le reste polymérique de rang p du reste G. Lorsque m et p sont des nombres décimaux, la formule (II) représente un mélange de composés :

$$b_1 \, H\text{-}G\text{-}O\text{-}R\text{-}O\text{-}G\text{-}H + b_2 \, H\text{-}G\text{-}O\text{-}R\text{-}O\text{-}(G)_2\text{-}H + b_3 \, H\text{-}G\text{-}O\text{-}R\text{-}O\text{-}(G)_3\text{-}H + ... + bq \, H\text{-}G\text{-}O\text{-}R\text{-}O\text{-}(G)_q\text{-}H +$$
$$c_1 \, H\text{-}(G)_2\text{-}O\text{-}R\text{-}O\text{-}G\text{-}H + c_2 \, H\text{-}(G)_2\text{-}O\text{-}R\text{-}O\text{-}(G)_2\text{-}H + c_3 \, H\text{-}(G)_2\text{-}O\text{-}R\text{-}O\text{-}(G)_3\text{-}H + ... \, cq \, H\text{-}(G)_2\text{-}O\text{-}R\text{-}O\text{-}(G)_q\text{-}H +... , d_1 \, H\text{-}(G)_3$$
$$\text{-}O\text{-}R\text{-}O\text{-}G\text{-}H + d_2 \, H\text{-}(G)_3\text{-}O\text{-}R\text{-}O\text{-}(G)_2\text{-}H + d_3 \, H\text{-}(G)_3\text{-}O\text{-}R\text{-}O\text{-}(G)_3\text{-}H + ... \, dq \, H\text{-}(G)_3\text{-}O\text{-}R\text{-}O\text{-}(G)_q\text{-}H + e_1 \, H\text{-}(G)_4\text{-}O\text{-}R\text{-}O\text{-}$$
$$G\text{-}H + e_2 \, H\text{-}(G)_4\text{-}O\text{-}R\text{-}O\text{-}(G)_2\text{-}H + e_3 \, H\text{-}(G)_4\text{-}O\text{-}R\text{-}O\text{-}(G)_3\text{-}H + ... \, eq \, H\text{-}(G)_4\text{-}O\text{-}R\text{-}O\text{-}(G)_q\text{-}H + f_1 \, H\text{-}(G)_5\text{-}O\text{-}R\text{-}O\text{-}G\text{-}H + f_2$$
$$H\text{-}(G)_5\text{-}O\text{-}R\text{-}O\text{-}(G)_2\text{-}H + f_3 \, H\text{-}(G)_5\text{-}O\text{-}R\text{-}O\text{-}(G)_3\text{-}H + ... \, fq \, H\text{-}(G)_5\text{-}O\text{-}R\text{-}O\text{-}(G)_q\text{-}H,$$

avec q représentant un nombre entier compris entre 1 et 5 et dans des proportions molaires telles que :

$$\sum_{q=5}^{q=1} b_q + \sum_{q=5}^{q=1} c_q + \sum_{q=5}^{q=1} d_q + \sum_{q=5}^{q=1} e_q + \sum_{q=5}^{q=1} f_q = 1 \text{ avec } b_1 > 0$$

Selon un aspect particulier de la présente invention, dans les formules (I) et (II), n, m et p indépendamment les uns des autres, sont compris entre 1,05 et 5, et plus particulièrement entre 1,05 et 2.

Par sucre réducteur, on désigne dans les formules (I) et (II), les dérivés saccharidiques qui ne présentent pas dans leurs structures de liaison glycosidique établie entre un carbone anomérique et l'oxygène d'un groupement acétal tels qu'ils sont définis dans l'ouvrage de référence : « Biochemistry », Daniel Voet/Judith G. Voet, p. 250, John Wyley & Sons, 1990. Les structures oligomériques (G)n, (G)m et (G)p, peuvent se présenter sous toute forme d'isomérie, qu'il s'agisse d'isomérie optique, d'isomérie géométrique ou d'isomérie de position ; elles peuvent aussi représenter un mélange d'isomères.

Dans la formule (I) telle que définie ci-dessus, le groupe HO-R-O est lié à G par le carbone anomérique du reste saccharide, de manière à former une fonction acétal.

Dans la formule (II) telle que définie ci-dessus, le groupe O-R-O est lié à chaque reste G par le carbone anomérique de chaque reste saccharide, de manière à former deux fonctions acétal distinctes.

Selon un aspect particulier de la présente invention, dans la définition des composés de formules (I) et (II), G représente un sucre réducteur choisi parmi le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le dextrane ou le tallose et plus particulièrement un sucre réducteur choisi parmi le glucose, le xylose ou l'arabinose.

Un tel système émulsionnant (A) peut être préparé par simple mélange des composés de formule (I), des composés de formule (II) et de 1,12-octadécanediol en des proportions prédéterminées souhaitées.

Un tel système émulsionnant (A) peut également être préparé selon un procédé comprenant :

Une étape a) de réaction du sucre réducteur G avec un excès stoechiométrique de 1,12-octadécanediol en présence d'un système catalytique acide.

Dans un tel procédé de préparation du système émulsionnant (A), l'étape a) est généralement mise en oeuvre dans un réacteur, en maîtrisant le rapport stoechiométrique entre les deux réactants, et par agitation mécanique dans des conditions de température et de vide partiel prédéterminées, par exemple à une température comprise entre 70°C et 130°C et sous un vide partiel compris entre 300 mbars (3 $10^4$ Pa) et 20 mbars (2 $10^3$ Pa).

Par système catalytique acide on désigne les acides forts comme l'acide sulfurique, l'acide chlorhydrique, l'acide phosphorique, l'acide nitrique, l'acide hypophosphoreux, l'acide méthanesulfonique, l'acide para-toluène sulfonique, l'acide trifluorométhane sulfonique, ou les résines échangeuses d'ions acides.

Un tel système émulsionnant (A) peut également être préparé selon une variante du procédé tel que décrit précédemment, comprenant :

Une étape a1) de réaction d'un sucre réducteur G avec un alcool de formule (III) :

$$R_3\text{-OH} \qquad \text{(III)},$$

dans laquelle $R_3$ représente un radical aliphatique linéaire comportant de 1 à 4 atomes de carbone, et plus particulièrement avec le butanol, en présence d'un système catalytique acide, pour former l'acétal de formule (IV) :

$$R_3\text{O-(G)p} \qquad \text{(IV)},$$

dans laquelle p représente un nombre décimal compris entre 1 à 5.

Une étape b) de trans-acétalisation de l'acétal de formule (IV) obtenu à l'étape a1), par ajout d'un excès 1,12-octadécanediol avec distillation sous vide de l'alcool de formule (III) formé in-situ.

L'étape a1) de la variante du procédé telle que définie ci-dessus, est plus particulièrement mise en oeuvre à une température comprise entre 90°C et 105°C, sous vide partiel, et elle souvent accompagnée de l'élimination concomitante de l'eau formée lors de la réaction. Comme système catalytique mis en oeuvre dans cette étape a1), il y a plus particulièrement celui tel que décrit pour la mise en oeuvre du procédé de préparation dont est issue ladite variante.

Le procédé et sa variante peuvent être, si nécessaire ou si désiré, complétés par des opérations de neutralisation, de filtration et de décoloration.

Le 1,12-octadécanediol utilisé pour la préparation du système émulsionnant (A) compris dans la composition objet de l'invention est un diol provenant de l'hydrogénation des acides gras de l'huile de ricin. Il est notamment commercialisé par la société Cognis sous l'appellation Speziol™ C18/2.

Par agent épaississant et/ou gélifiant d'origine naturelle, on désigne les substances chimiques permettant d'épaississir des phases aqueuses, dont les matières premières nécessaires à leur préparation sont des végétaux, des algues, des minéraux, et les substances chimiques qui sont obtenues par action fermentive d'au moins une bactérie et d'au moins un substrat hydrocarboné dont les matières premières nécessaires à leur préparation sont des végétaux ou des algues.

Parmi les agents épaississants et/ou gélifiants d'origine naturelle, on désigne des polysaccharides et des protéines topiquement acceptables et habituellement utilisés dans ce domaine d'activité, comme par exemple la gomme d'acacia, la gomme de karaya, la gomme de ghatti, la gomme de sclerotium, la gomme de guar, le caroube, la pectine, le fenugreek, les carraghénates, les alginates, les galactomannanes, la cellulose et ses dérivés, l'amidon et ses dérivés, la gomme de xanthane, les silicates d'aluminium, les silicates de magnésium.

Selon un aspect particulier, la composition objet de la présente invention, comprend pour 100% de sa masse entre 0,01% et 5% massique d'au moins un agent épaississant et/ou gélifiant choisi parmi les agents épaississants et/ou gélifiants d'origine naturelle, et plus particulièrement entre 0,1% et 5% massique.

Selon un autre aspect particulier, la composition objet de la présente invention comprend au moins un agent épaississant et/ou gélifiant d'origine naturelle choisi parmi les éléments d'un groupe constitué par la gomme de xanthane, la gomme d'acacia, la gomme de guar, la pectine, le caroube, le fenugreek, les carraghénates, les alginates et les galactomannanes.

Par huiles d'origine végétale à base de monoglycérides et/ou de diglycérides et/ou de triglycérides, on désigne les substances chimiques comprenant des monoglycérides de formule (A1) et/ou de formule (A'1), et/ou des diglycérides de formule (A2) et/ou de formule (A'2), et/ou des triglycérides de formule (A3) dont les matières premières nécessaires à leur préparation sont des végétaux :

$$CH_3\text{-}(CH_2)_{x1}\text{-C(O)-O-}CH_2\text{-CH(OH)-}CH_2\text{-OH} \qquad \text{(A1)}$$

$$HO\text{-}CH_2\text{-CH[O-C(O)-}(CH_2)_{x2}\text{-}CH_3]\text{-}CH_2\text{-O-H} \qquad \text{(A'1)}$$

$$CH_3\text{-}(CH_2)_{x3}\text{-}C(O)\text{-}O\text{-}CH_2\text{-}CH(OH)\text{-}CH_2\text{-}O\text{-}C(O)\text{-}(CH_2)_{x4}\text{-}CH_3 \qquad (A2)$$

$$CH_3\text{-}(CH_2)_{x5}\text{-}C(O)\text{-}O\text{-}CH_2\text{-}CH[O\text{-}C(O)\text{-}(CH_2)_{x6}\text{-}CH_3]\text{-}CH_2\text{-}O\text{-}H \qquad (A'2)$$

$$CH_3\text{-}(CH_2)_{x7}\text{-}C(O)\text{-}O\text{-}CH_2\text{-}CH[O\text{-}C(O)\text{-}(CH_2)_{x8}\text{-}CH_3]\text{-}CH_2\text{-}O\text{-}C(O)\text{-}(CH_2)_{x9}\text{-}CH_3 \quad (A3)$$

avec x1, x2, x3, x4, x5, x6, x7, x8 et x9, identiques ou différents, représentent un nombre entier compris entre 7 et 23.

Parmi les huiles d'origine végétale à base de monoglycérides et/ou de ditriglycérides et/ou de triglycérides telles que définies précédemment, on peut citer par exemple l'huile d'amandes douces, l'huile de coprah, l'huile de ricin, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile de pastel, l'huile de bourrache, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyaux d'abricot, l'huile de calophyllum, l'huile de sysymbrium, l'huile d'avocat, l'huile de calendula, l'huile de chanvre, les huiles issues de fleurs, les huiles issues de légumes, les triglycérides obtenus par réaction d'estérification entre un acide gras d'origine végétale et le glycérol comme par exemple le caprylic capric triglycéride commercialisé sous l'appellation DUB MCT par la société STEARINERIE DUBOIS, les triglycérides obtenus par réaction d'estérification entre les acides gras comportant sept atomes de carbone et le glycérol commercialisé sous l'appellation DUB THG par par la société STEARINERIE DUBOIS, les triglycérides obtenus par réaction d'estérification entre les acides gras comportant 22 atomes de carbone et le glycérol commercialisé sous l'appellation SYNCHROWAX HRC par la société CRODA.

L'expression « cosmétiquement acceptable » utilisée dans la définition de la phase aqueuse (P1) de la composition se présentant sous la forme d'une émulsion huile-dans-eau objet de la présente invention, signifie selon la directive du Conseil de la Communauté Economique Européenne N°76/768/CEE du 27 juillet 1976 modifiée par la directive N°93/35/CEE du 14 juin 1993, que ladite phase aqueuse (P1) comprend de l'eau et toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

Une phase aqueuse (P1) cosmétiquement acceptable comprise dans ces compositions se présentant sous la forme d'une émulsion huile-dans-eau objet de la présente invention contient de l'eau, et peut contenir classiquement un ou plusieurs solvants organique cosmétiquement acceptables, un mélange d'eau et d'un ou plusieurs solvants organiques cosmétiquement acceptables. Les solvants cosmétiquement acceptables peuvent plus particulièrement être choisis parmi les alcools polyhydriques comme par exemple le glycérol, le diglycérol, les oligomères du glycérol, l'éthylène glycol, le propylène glycol, le butylène glycol, l'hexylène glycol, le diéthylène glycol, le xylitol, l'érythritol, le sorbitol, ou les alcools hydrosolubles tels que l'éthanol, l'isopropanol ou le butanol.

Selon un autre aspect plus particulier, la composition objet de la présente invention comprend, pour 100% de sa masse, de 0,5% massique à 10% massique de dihydroxyacétone.

La dihydroxyacétone est un produit couramment utilisé en cosmétique comme un agent de bronzage et/ou de brunissement artificiel de la peau qui permet d'obtenir un effet de bronzage ou de brunissement d'apparence plus ou moins semblable a celui qui peut résulter d'une exposition prolongée au soleil ou sous une lampe à rayonnement ultraviolet. La composition se présentant sous la forme d'une émulsion huile-dans-eau comprenant pour 100% de sa masse de 0,5% a 10% massique de dihydroxyacétone et objet de la présente invention peut également être associée à d'autres agents de bronzage et/ou de brunissement de la peau parmi lesquels on peut citer les composés mono- ou polycarbonylés comme par exemple l'isatinne, l'alloxane, la n inhydrine, le glycéraldehyde, l'aldéhyde mésotartrique, la glutaraldéhyde, l'érythrulose.

De façon générale, la composition objet de la présente invention, comporte également des excipients et/ou des principes actifs habituellement mis en oeuvre dans le domaine des formulations cosmétiques, dermocosmétiques, pharmaceutiques ou dermopharmaceutiques, comme des agents tensioactifs co-émulsionnants, comme des agents tensioactifs détergents, comme des agents stabilisants, des composés filmogènes, des agents nacrants, des agents surgraissants, des tensioactifs épaississants et/ou gélifiants, des agents séquestrants, des agents hydrotropes, des agents plastifiants, des agents surgraissants, des anti-oxydants, des corps gras d'origine naturelle, des agents conservateurs, des principes actifs destinés à apporter une action traitante vis a vis de la peau ou des cheveux, des filtres solaires, des charges minérales ou des pigments, des particules procurant un effet visuel ou destinées a l'encapsulation d'actifs, des particules exfoliantes, des agents de texture.

Parmi les principes actifs pouvant être compris dans la composition objet de la présente invention, on peut citer les principes actifs ayant une action éclaircissante ou dépigmentante, une action hydratante, une action tenseur, une action apaisante ou relaxante, une action anti inflammatoire, une action amincissante, une action lipolytique, une action drainante, une action détoxifiante, une action une action énergisante, décontractante, une action stimulante, une action émoliente, une action neuromodulatrice, une action protectrice, une action purifiante, séborégulatrice, antichute, une action anti-age, une action raffermissante, restructurante, antiradicalaire, ou antioxydante. De tels principes actifs sont par exemple les protéines N-acylées, les peptides N-acylés comme par exemple le MATRIXIL™, les acides aminés N-acylés se présentant sous leurs formes acides et/ou salifiées comme par exemple le N-(ω-undécylènoyl) phénylalanine commercialisé par la société SEPPIC sous l'appellation SEPIWHITE™MSH, l'octanoylglycine commercialisé par la société SEPPIC sous l'appellation LIPACIDE™C8G, l'undécylénoylglycine commercialisé par la société SEPPIC sous l'appellation LIPACIDE™UG, les hydrolysâts partiels de protéines N-acylés, les acides aminés, les peptides, les hydrolysats totaux de protéines, les vitamines liposolubles, les dérivés de vitamines liposolubles comme par exemple le Rétinol, la vitamine E et ses dérivés, les vitamines hydrosolubles et/ou hydrodispersibles comme par exemple la Vitamine C et ses dérivés, le magnésium ascorbyl phosphate et ses dérivés, l'ascorbyl glucoside, l'acide phytique, les acides de fruits, les extraits aqueux ou hydroalcooliques ou hydroglycoliques de polyphénols, de quinoa, de panais, de potentille, de raisin, de vin, les extraits d'olives, les extraits de marc, les polyols (par exemple, la glycérine ou le butylène glycol), les dérivés de lait, ou les différents composants entrant dans la composition du NMF (natural moisturizing factor) par exemples l'urée, l'acide pyrrolidone carboxylique ou les dérivés de cet acide, les acides aminés, les sels minéraux, les sucres ou les dérivés des sucres, les polysaccharides ou leurs dérivés, les hydroxyacides par exemple l'acide lactique, les extraits végétaux aqueux ou hydroalcooliques ou hydroglycoliques, tels que les extraits végétaux riches en tanins, les extraits végétaux riches en isoflavones ou les extraits végétaux riches en terpènes, les extraits d'algues d'eau douce ou marines, les extraits marins en général comme les coraux, les extraits bactériens ; les minéraux comme les GIVOBIO™, les dérivés de calcium, de magnésium, de cuivre, de cobalt, de zinc, de lithium, ou de manganèse, les sels d'argent ou d'or ; les actifs ayant une propriété énergisante ou stimulante comme le SEPITONIC™ M3 ou le Physiogényl™ ; les actifs auto-bronzants comme par exemple la dihydroxyacétone et l'érythrulose ; les actifs hydratant ou restructurant de l'épiderme comme par exemple l'AQUAXYL™, les lipides en général, les lipides tels que les céramides ou les phospholipides, les actifs ayant une action amincissante ou lipolytique comme la caféine ou ses dérivés, le panthénol et ses dérivés comme le SEPICAP™ MP, les actifs anti-age comme le SEPILIFT™ DPHP, le LIPACIDE™ PVB, les actifs augmentant la synthèse des composants de la matrice extracellulaire par exemple, le collagène, les élastines, les glycosaminoglycanes, les actifs agissant favorablement sur la communication cellulaire chimique comme les cytokines ou physiques comme les intégrines, les actifs créant une sensation de « chauffe » sur la peau comme les activateurs de la microcirculation cutanée (exemple des nicotinates) ou des produits créant une sensation de « fraîcheur » sur la peau (exemple du menthol et de ses dérivés) ; le miel ; les eaux florales comme par exemple l'eau de jeunes pousses d'orge.

Comme exemples d'agents de texture éventuellement présents dans la composition objet de la présente invention, on peut citer des dérivés N-acylés d'acides aminés, comme par exemple la lauroyl lysine commercialisée sous l'appellation AMINOHOPE™LL par la société AJINOMOTO, l'octenyl starch succinate commercialisé sous l'appellation DRYFLO™ par la société NATIONAL STARCH, le myristyl polyglucoside commercialisé par SEPPIC sous l'appellation MONTANOV 14, les fibres de cellulose, les fibres de coton, les fibres de chitosane, le talc, la séricite, le mica.

Comme exemples d'agents opacifiants et/ou nacrants éventuellement présents dans la composition objet de la présente invention, on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras.

Comme exemples de tensioactifs épaississants et/ou gélifiants éventuellement présents dans la composition objet de la présente invention, on peut citer :

- les esters gras d'alkylpolyglycosides éventuellement alkoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120 ;
- les esters gras alkoxylés tels que le PEG 150 pentaérythrytyl tétrastéarate commercialisé sous l'appellation CROTHIX™ DS53, le PEG 55 propylene glycol oléate commercialisé sous l'appellation ANTIL™ 141 ;
- les carbamates de polyalkylène glycols à chaînes grasses tels que le PPG 14 laureth isophoryl dicarbamate commercialisé sous l'appellation ELFACOS™ T211, le PPG 14 palmeth 60 hexyl dicarbamate commercialisé sous l'appellation ELFACOS™ GT2125.

Comme exemples d'agents tensioactifs co-émulsionnants éventuellement présents dans la composition objet de la présente invention, on peut citer les tensioactifs non ioniques, les tensioactifs anioniques, les tensioactifs cationiques.

Comme exemples d'agents tensioactifs non ioniques co-émulsionnants éventuellement présents dans la composition objet de la présente invention, on peut citer les esters d'acides gras et de sorbitol, comme par exemple les produits commercialisés sous les appellations MONTANE™80 et MONTANE™85 et MONTAN™60 par la société SEPPIC ; les alkylpolyglycosides ; les compositions d'alkylpolyglycosides et d'alcools gras linéaires ou ramifiés, comme par exemple les compositions commercialisées sous les appellations MONTANOV™ et FLUIDANOV™ par la société SEPPIC ; les esters d'acide gras et de polyglycérol, comme par exemple les produits commercialisés sous la dénomination ISOLAN™ GI34 par la société BASF et PLUROL™ DIISOSTEARIQUE par la société GATTEFOSSE ; l'huile de ricin éthoxylée et l'huile de ricin hydrogénée éthoxylée comme par exemple le produit commercialisé sous la dénomination SIMULSOL™ 989 par la société SEPPIC ; les compositions comprenant du stéarate de glycérol et de d'acide stéarique éthoxylé entre 5 moles et 150 moles d'oxyde d'éthylène, comme par exemple la composition comprenant de l'acide stéarique éthoxylé à 135 moles d'oxyde d'éthylène et du stéarate de glycérol commercialisée sous l'appellation SIMULSOL 165 par la société SEPPIC ; les polyhydroxystéarates de polyglycol ou de polyglycérol comme par exemple les produits dénommés HYPERMER™ B246, ARLACEL™P135 commercialisés par la société UNIQEMA, le produit dénommé DEHYMULS™ PGPH commercialisé par la société COGNIS, le produit dénommé DECAGLYN™ 5HS commercialisé parla société NIKKO ; les copolymères polyéthylèneglycol-alkylglycol comme le PEG-45 dodécylglycol copolymère tel que le produit commercialisé sous la dénomination ELFACOS™ ST 9 par la société AKZO ; les esters de sorbitan éthoxylés comme par exemple les produits commercialisés sous la dénomination MONTANOX™ par la société SEPPIC ; les esters de mannitan ; les esters de mannitan éthoxylés ; les esters de sucrose ; les esters de méthylglucoside.

Comme exemples d'agents tensioactifs anioniques co-émulsionnants éventuellement présents dans la composition objet de la présente invention, on peut citer le décylphosphate, le cétylphosphate commercialisé sous l'appellation AMPHISOL™ par la société DSM, le glycéryl stéarate citrate ; le cétéarylsulfate ; la composition arachidyl/béhényl phosphates et arachidyl/béhényl alcools commercialisée sous l'appellation SENSANOV™WR par la société SEPPIC ; les savons comme par exemple le stéarate de sodium ou le stéarate de triéthanolammonium, les lipoaminoacides salifiés comme par exemple le stéaroyl glutamate.

Comme exemples d'agents tensioactifs cationique co-émulsionnants éventuellement présents dans la composition objet de la présente invention, on peut citer les aminoxydes, le quaternium-82 et les tensioactifs décrits dans la demande de brevet WO96/00719 et principalement ceux dont la chaîne grasse comprend au moins 16 atomes de carbone.

Comme exemples d'agents tensioactifs détergents éventuellement présents dans la composition objet de la présente invention, on peut citer les tensioactifs détergents anioniques, les tenseioctfis détergents cationiques, les tensioactifs détergents amphotères, les tensioactifs détergents non ioniques.

Parmi les tensioactifs détergents anioniques éventuellement présents dans la composition objet de la présente invention, on peut citer particulièrement les sels de métaux alcalins, les sels de métaux alcalinoterreux, les sels d'ammonium, les sels d'amines, les sels d'aminoalcools des composés suivants : les alkyléthers sulfates, les alkylsulfates, les alkylamidoéthersulfates, les alkylarylpolyéthersulfates, les monoglycérides sulfates, les alpha-oléfinesulfonates, les paraffines sulfonates, les alkylphosphates, les alkylétherphosphates, les alkylsulfonates, les alkylamidesulfonates, les alkylarylsulfonates, les alkylcarboxylates, les alkylsulfosuccinates, les alkyléthersulfosuccinates, les alkylamidesulfosuccinates, les alkylsulfoacétates, les alkylsarcosinates, les acyliséthionates, les N-acyltaurates, les acyllactylates. Parmi les tensioactifs anioniques, on citera également les lipoaminoacides, les lipoprotéines, les lipopeptides, les dérivés des lipoprotéines, les dérivés de protéines, les sels d'acides gras, les sels d'acides d'huile de coprah éventuellement hydrogénée.

Parmi les tensioactifs détergents amphotères éventuellement présents dans la composition objet de la présente invention, on peut citer particulièrement les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs détergents cationiques éventuellement présents dans la composition objet de la présente invention, on peut citer particulièrement les dérivés d'ammoniums quaternaires.

Parmi les tensioactifs détergents non-ioniques éventuellement présents dans la composition objet de la présente invention, on peut citer particulièrement les alkylpolyglycosides, les dérivés d'huile de ricin, les polysorbates, les amides de coprah, les N-alkylamines.

Comme exemples de corps gras d'origine naturelle éventuellement présents dans la composition objet de la présente invention, on peut citer les alcools gras saturés ou insaturés, linéaires ou ramifiés d'origine végétale ; les acides gras saturés ou insaturés, linéaires ou ramifiés d'origine végétale ; les esters d'alcools gras et d'acides gras saturés ou insaturés, linéaires ou ramifiés d'origine végétale, comme par exemple le butyl stearate, le cetyl palmitate, le cetyl ricinoleate, le decyl oleate, le diisopropyl sebacate, le diethylhexyl sebacaté, l'éthyl linoleate, le trilaurin, l'isocetyl stearate, l'isopropyl palmitate, l'isopropyl myristate, l'isostearyl isostearate, le myristyl myristate, le neopentylglycol diheptanoate, l'éthylhexyl cocoate, l'éthylhexyl hydroxystearate, l'éthylhexyl palmitate, l'éthylhexyl stearate, l'octyldodecyl myristate, le pentaerylthrityl tétraisostearate, le propylene glycol dicaprylate/dicaprate, le stéaryl caprylate, le stéaryl heptanoate, la triisostearine, le béhényl béhénate, l'octyldodécyl stéaroyl stéarate, l'isocétyl stéaroyl stéarate, le pentaérythrityl tétrabéhénate, le pentaérylthrityl tétracaprylate/tétracaprate, le dipentaérythrityl hexacaprylate/caprate ; les éthers d'alcools gras et d'acides gras saturés ou insaturés, linéaires ou ramifiés d'origine végétale ; les alcanes linéaires ou ramifiés d'origine végétale, comme par exemple le phytosqualane; les alcènes linéaires ou ramifiés d'origine végétale, comme par exemple le phytosqualène ; les huiles végétales éthoxylées ; les esters méthyliques d'origine végétale éthoxylés ; les cires d'origine végétale, comme par exemple la cire de carnauba, la cire de candelilla, la cire de fibre de liège, la cire de canne à sucre, et la cire d'abeille.

Comme exemples de pigments éventuellement présents dans la composition objet de la présente invention, on peut citer le dioxyde de titane, l'oxychlorure de bismuth, les oxydes de fer marrons, les oxydes de fer jaune, les oxydes de fer noir, ou les oxydes de fer rouges ou encore les pigments nacrés blancs ou colorés tels que les Mica-Titane.

Comme filtres solaires éventuellement présents dans la composition objet de la présente invention, on peut citer tous ceux figurant dans la directive cosmétique 76/768/CEE modifiée annexe VII, et plus particulièrement les écrans solaires d'origine naturelle comme par exemple l'oxyde de titane et l'oxyde de zinc.

La composition objet de la présente invention, peut se présenter sous la forme de crèmes, de laits, de gels crèmes, de lotions fluides, de lotions fluides vaporisables. Lorsque la composition objet de la présente invention possède des caractéristiques de fluidité appropriées, elle peut aussi servir pour l'imprégnation de supports constitués de fibres textiles synthétiques ou naturelles, tissées ou non tissées, ou de papiers, pour constituer des articles, comme par exemple des lingettes, destinés au soin, à la protection ou au nettoyage de la peau, du cuir chevelu ou des cheveux, ou comme par exemple des papiers à usage sanitaire ou domestique.

La composition objet de la présente invention, peut être mise en oeuvre par application sur la peau, les cheveux ou le cuir chevelu, qu'il s'agisse d'une application directe dans le cas d'une composition cosmétique, dermocosmétique, dermopharmaceutique ou pharmaceutique, ou d'une application indirecte dans le cas d'un produit de soin, de protection, de nettoyage du corps se présentant sous la forme d'un article en textile, comme par exemple une lingette, ou en papier, comme par exemple un papier à usage sanitaire, destinés à être en contact avec la peau, les cheveux ou le cuir chevelu.

L'invention a aussi pour objet l'utilisation cosmétique de la composition telle que définie précédemment pour le nettoyage, pour la protection et/ou pour le soin de la peau, des cheveux ou du cuir chevelu.

La composition objet de la présente invention peut être utilisée pour le nettoyage de la peau, des cheveux ou du cuir chevelu, et plus particulièrement être utilisée comme gel pour le bain ou pour la douche, comme shampoing. Dans cette utilisation particulière, elle comprend en outre au moins un tensioactif détergent tel que décrit précédemment.

La composition objet de la présente invention, peut être utilisée pour le soin ou pour la protection de la peau, comme par exemple comme crème, comme lait ou comme lotion pour le soin ou pour la protection du visage, des mains et du corps.

La composition objet de la présente invention, peut aussi être utilisée comme produit protecteur de la peau contre les rayons du soleils, et comme produit de maquillage de la peau.

Selon un autre aspect particulier, l'invention a pour objet l'utilisation cosmétique de la composition telle que définié précedemment et comprenant, en outre pour 100% de sa masse, de 0,5% massique à 10% massique de dihydroxyacétone, pour bronzer et/ou brunir artificiellement la peau.

L'invention a aussi pour objet un procédé de traitement cosmétique pour bronzer et/ou brunir artificiellement la peau, caractérisée par le fait qu'il consiste à appliquer sur celle-ci la composition telle que définie précédemment et comprennant en outre pour 100% de sa masse, de 0,5% massique à 10% massique de dihydroxyacétone.

Selon un autre aspect, la présente invention a pour objet un procédé de préparation de la composition telle que définie précédemment, comprenant :

au moins une étape a) de mélange de la phase grasse (P2) avec le système émulsionnant (A), tel que décrit précédemment ;
au moins une étape b) d'émulsification du mélange de la phase grasse (P2) et du système émulsionnant (A) obtenu à l'issue de l'étape a) avec la phase aqueuse (P1).

Dans le procédé objet de l'invention, la phase grasse (P2) comprend une ou plusieurs huiles végétales à base de monoglycérides et/ou de diglycérides et/ou de triglycérides, telles que définies précédemment. Dans le cas où la phase grasse (P2) n'est pas constituée par une seule huile végétale à base de monoglycérides et/ou de diglycérides et/ou de triglycérides, la phase grasse (P2) est préparée par mélange des ingrédients la constituant à une température typiquement comprise entre 20°C et 85°C, et encore plus particulièrement à une température comprise entre 20°C et 60°C, et par le moyen de tout dispositif de mélange connu de l'homme du métier, comme par exemple par le moyen d'un dispositif d'agitation mécanique équipé d'un mobile de type « ancre », à des vitesses d'agitation comprises entre 50 tours/minute et 500 tours/minutes, plus particulièrement entre 50 tours/minute et 300 tours/minutes.

Dans le procédé objet de l'invention tel que décrit ci-dessus, l'étape a) de mélange de la phase grasse (P2) avec le système émulsionnant (A) peut être avantageusement mise en oeuvre à une temperature inférieure ou égale a 90°C, plus particulierement à une temperature comprise entre 50°C et 85°C, et encore plus particulierement à une temperature comprise entre 60°C et 85°C.

L'étape a) de mélange de la phase grasse (P2) avec le système émulsionnant (A) du procédé objet de l'invention peut être réalisée par le moyen de tout dispositif de mélange connu de l'homme du métier, comme par exemple le moyen d'un dispositif d'agitation mécanique équipé d'un mobile de type « ancre », à des vitesses d'agitation comprises entre 50 tours/minute et 500 tours/minutes, plus particulièrement entre 50 tours/minute et 300 tours/minutes, et comme par exemple par le moyen d'un dispositif d'agitation de type rotor-stator à des vitesses d'agitation comprises entre 100 tours/minute et 10 000 tours/minutes, plus particulièrement entre 500 tours/minutes et 4000 tours/minutes.

Dans le procédé objet de l'invention, l'étape b) d'émulsification du mélange de la phase grasse (P2) et du système émulsionnant (A), tel que préparé à l'issu de l'étape a), avec la phase aqueuse (P1) peut être avantageusement mise en oeuvre à une temperature comprise entre 20°C et 90°C, plus particulièrement à une température comprise entre 50°C et 85°C, et encore plus particulièrement à une température comprise entre 60°C et 85°C.

Dans le procédé objet de l'invention, l'étape b) d'émulsification peut être réalisée par le moyen de tout dispositif de mélange connu de l'homme du métier, comme par exemple le moyen d'un dispositif d'agitation mécanique équipé d'un mobile de type « ancre », à des vitesses d'agitation comprises entre 50 tours/minute et 500 tours/minutes, plus particulièrement entre 50 tours/minute et 300 tours/minutes, et comme par exemple par le moyen d'un dispositif d'agitation de type rotor-stator à des vitesses d'agitation comprises entre 100 tours/minute et 10 000 tours/minutes, plus particulièrement entre 500 tours/minutes et 4000 tours/minutes.

Dans le procédé objet de l'invention tel que décrit ci-dessus, la phase aqueuse (P1) comprend de l'eau, et éventuellement un ou plusieurs solvants organiques cosmétiquement acceptables tels que décrits précédemment, et au moins un agent épaississant et/ou gélifiant d'origine naturelle tel que décrit précédemment. La phase aqueuse (P1) est préparée par mélange de l'eau, et éventuellement d'un ou plusieurs solvants organiques cosmétiquement acceptable, avec au moins un agent épaississant et/ou gélifiant d'origine naturelle tel que décrit précédemment, à une température comprise entre 20°C et 85°C, et encore plus particulièrement à une température comprise entre 20°C et 60°C, et par le moyen de tout dispositif de mélange connu de l'homme du métier, comme par exemple par le moyen d'un dispositif d'agitation mécanique équipé d'un mobile de type « ancre », à des vitesses d'agitation comprises entre 50 tours/minute et 500 tours/minutes, plus particulièrement entre 50 tours/minute et 300 tours/minutes.

Les exemples suivants illustrent l'invention, sans toutefois la limiter.

## I - Préparation de compositions sous la forme d'une émulsion huile dans eau stable selon l'invention

### I - 1 - Préparation d'une composition émulsionnante (A1) constituée d'un mélange des produits de réaction du glucose et du 1,12-octadécanediol et de 1,12-octadécanediol.

On introduit 1074,6 g de 1,12-octadécanediol dans un réacteur en verre à double enveloppe, dans laquelle circule un fluide caloporteur, et muni d'une agitation efficace. Le 1,12-octadécanediol est fondu à une température de 90°C en 30 minutes, et 100,3 g de glucose anhydre sont dispersés progressivement dans le milieu réactionnel et homogénéisés à 90°C pendant 20 minutes. On ajoute alors au mélange ainsi obtenu un système catalytique acide constitué de 1,50 g d'acide sulfurique à 96%. Le milieu réactionnel est ensuite placé sous un vide partiel de 90 mbars à 25 mbars, et maintenu à une température de 103°C - 106°C pendant une durée de six heures avec évacuation de l'eau formée au moyen d'un montage de distillation. Le milieu réactionnel est ensuite refroidi à 95°C-100°C et neutralisé par ajout de 1,47 g de soude à 30% , pour amener le pH d'une solution à 5% de ce mélange à une valeur comprise entre 5,5 et 7,5. La composition (A1) ainsi obtenue est ensuite vidangée.

Les caractéristiques analytiques de la composition (A1) sont les suivantes :

Aspect (visuel) : cire beige à température ambiante ;
pH solution à 5% : 7,3 ;
Point de fusion : 70,3°C
Teneur résiduelle en eau : 0,21%
Teneur en 1,12-octadécanediol 81,4%
Teneur en alkylpolyglucosides de 1,12-octadécanediol : 18,2%

### I - 2 - Préparation d'émulsions cosmétiques huile-dans-eau stables.

Les émulsions (E1), (E'1), (E2), (E'2) et (E3) sont préparées selon le même mode opératoire suivant :

- les composants de la phase grasse sont introduits successivement dans un réacteur et le mélange résultant est porté par l'intermédiaire d'un agitateur muni d'un mobile de type ancre, à une vitesse de 80 tours/minute à une température de 80°C ;
- le système émulsionnant est alors ajouté sur la phase grasse à une température de 80°C, et le mélange obtenu est homogénéisé pendant 30 minutes par l'intermédiaire d'un agitateur muni d'un mobile de type ancre, à une vitesse de 80 tours/minute ;

- la phase aqueuse comprenant l'eau, un agent conservateur, un agent complexant (le DISSOLVINE™GL-38) et un agent épaississant (gomme xanthane commercialisée sous le nom KELTROL™ CG T ou un agent épaississant à base de copolymères acryliques commercialisé sous le nom CAPIGEL™98) est préparée séparément par mélange de ses différents ingrédients à une température de 25°C sous agitation mécanique par l'intermédiaire d'un agitateur muni d'un mobile de type ancre, à une vitesse de 80 tours/minutes ;

- la phase aqueuse est ensuite portée à une température de 80°C, maintenue sous agitation mécanique par l'intermédiaire de l'agitateur mécanique muni d'un mobile ancre à une vitesse de 80 tours/minutes, puis ajoutée sur le mélange comprenant la phase grasse et le système émulsionnant à une température de 80°C ;

- le mélange résultant est cisaillée au moyen d'un émulseur de type rotor-stator, commercialisé par la société SILVERSON, pendant une durée de 4 minutes à une vitesse de 4000 tours/minute, puis refroidit à une température de 30°C, sous agitation mécanique par l'intermédiaire de l'agitateur mécanique muni d'un mobile ancre à une vitesse de 80 tours/minutes sous ancre pendant une durée de 10 minutes ;

- l'agent conservateur (le GEOGARD™™ 221) est ensuite introduit sur le mélange émulsionné ainsi obtenu, qui est ensuite refroidi à température ambiante et agité pendant une durée supplémentaire de 10 minutes à l'aide d'un agitateur muni d'un mobile de type ancre à une vitesse de 80 tours/minutes.

Les compositions massiques de chacune des émulsions sont consignées dans le tableau 1 ci-dessous.

**Tableau 1**

|  | (E1) | (E'1) | (E'2) | (E2) | (E3) |
|---|---|---|---|---|---|
| ***Système émulsionnant*** |  |  |  |  |  |
| - Composition (A1) | 4% | 4% | 4% | 0% | 0% |
| - MONTANOV™ 68[1] | 0% | 0% | 0% | 4% | 0% |
| - ARLATONE™ 2121[2] | 0% | 0% | 0% | 0% | 4% |
| ***Phase grasse*** |  |  |  |  |  |
| LANOL™ 2681[3] | 10% | 10% | 10% | 10% | 10% |
| Huile d'Amandes douces | 10% | 0% | 0% | 10% | 10% |
| Huile de jojoba | 0% | 10% | 10% | 0% | 0% |
| DERMOFEEL™™ Toco 70[7] | 0,05% | 0,05% | 0,05% | 0,05% | 0,05% |
| ***Phase aqueuse*** |  |  |  |  |  |
| GEOGARD™™ 221[4] | 0,6% | 0,6% | 0,6% | 0,6% | 0,6% |
| DISSOLVINE™GL-38[5] | 0,3% | 0,3% | 0,3% | 0,3% | 0,3% |
| Acide citrique | Qs pH=5 | Qs pH=5 | Qs pH = 5 | Qs pH = 5 | Qs pH = 5 |
| Eau | Qsp 100% | Qsp 100% | Qsp100% | Qsp 100% | Qsp 100% |
| KELTROL™ CG T[6] | 0,3% | 0% | 0,3% | 0,3% | 0,3% |
| CAPIGEL™98[8] | 0% | 0,3% | 0% | 0% | 0% |
| (1) : Mélange cétéaryl glucoside / Alcool cétéarylique tel que décrit dans WO 92/06778 |||||
| (2) : Mélange stéarate de sorbitan/cocoate de sucrose |||||
| (3) : Ester gras (coco-caprylate caprate) |||||
| (4) : Mélange d'acide dehydroacétique et d'alcool benzylique utilisé comme agent conservateur |||||
| (5) : Diacétate de glutamate tétrasodique utilisé comme agent complexant |||||
| (6) : Gomme de xanthane |||||
| (7) : Mélange de tocophérols et d'huile de graines d'Hélianthus Annus utilisé comme antioxydant |||||
| (8) : Agent épaississant à base de copolymères acryliques |||||

Les émulsions ainsi préparées sont conservées dans une enceinte climatique isolée et régulée à une température de 20°C pendant 7 jours. A l'issue de cette période de 7 jours, l'aspect de chaque émulsion est observé et sa viscosité mesurée (Brookfield LVT, mobile 4, vitesse 6 ; en mPa.s). Les émulsions sont ensuite replacées et conservées dans la même enceinte climatique isolée et régulée à une température de 20°C jusqu'à 1 mois. Après une période d'un mois, chaque émulsion est sortie de l'enceinte climatique pour en observer l'aspect.

Les résultats des observations et des mesures de viscosités obtenus sont consignés dans le tableau 2 suivant :

**Tableau 2**

|  | (E1) | (E'1) | (E'2) | (E2) | (E3) |
|---|---|---|---|---|---|
| Aspect à 7 jours à 20°C | Homogène | Homogène | Homogène | Homogène | Homogène |
| Viscosité à 7 jours à 20°C | 30.000 | 30.000 | 31.000 | 27.000 | 20.000 |
| Aspect à 1 mois à 20°C | Homogène | Homogène | Homogène | Homogène | Homogène |

Ces résultats font apparaître que l'émulsion huile-dans-eau (E1) selon l'invention reste homogène au stockage à température ambiante après une durée minimale d'un mois, tout comme les émulsions huile-dans-eau (E'1), (E2), (E'2) et (E3) de l'état de la technique.

**II - Mise en évidence de l'amélioration de l'effet « savonnant » d'une composition à usage topique selon l'invention.**

**II -** 1 - **Evaluation de l'effet "savonnant"**

L'effet « savonnant » se traduit par l'apparation sur la surface de la peau d'un film blanchâtre lors de l'application de la composition sur la peau. Son évaluation est déterminée expérimentalement par un panel représentatif d'utilisateurs dûment entrainés, selon le protocole détaillé au paragraphe b) suivant.

a) - Principe de la méthode

L'effet "savonnant" d'une composition sur la peau est évalué lors d'une opération d'application sur la peau par un panel de 11 personnes, dûment entrainées à cette évaluation, qui notent le nombre de rotations effectuées par les doigts appliquant la composition sur la peau à partir duquel un film blanchâtre apparait.

b) - Protocole expérimental

La surface de la peau de l'expérimentateur sur laquelle doit être appliquée la composition à évaluer est préalablement nettoyée avec de l'éthanol puis séchée à l'aide d'un papier absorbant. 50 $\mu$l de composition à évaluer sont prélevés à l'aide d'une micropipette puis déposés sur la peau de l'intérieur du poignet de l'expérimentateur. L'expérimentateur effectue ensuite des rotations d'environ trois centimètres avec deux doigts (index et majeur) de son autre main avec une fréquence d'environ une rotation par seconde. L'expérimentateur note le nombre de rotations effectuées jusqu'à l'apparition d'un film blanchâtre sur la surface de la peau sur laquelle a été appliquée la composition.

c) - Expression des résultats

Lorsque le nombre de rotations nécessaire à l'apparition d'un film blanchâtre sur la peau de l'expérimentateur est compris entre 1 et 30, on considère que la composition testée est "savonnante".
Lorsque le nombre de rotations nécessaire à l'apparition d'un film blanchâtre sur la peau de l'expérimentateur est compris entre 31 et 60, on considère que la composition testée est "peu savonnante".
Lorsque le nombre de rotations nécessaire à l'apparition d'un film blanchâtre sur la peau de l'expérimentateur est supérieur à 61, on considère que la composition testée est "non savonnante".
Pour chaque composition testée, on relève le nombre de rotations ainsi noté par chacun des 11 expérimentateurs et on calcule ensuite la moyenne arithmétique selon la formule suivante :

Nombre de rotations moyen = Somme des nombres de rotations de chaque expérimentateur/11.

**II - 2 - Evaluation de la consistance d'une composition.**

Par consistance d'une composition, on entend la résistance à l'écoulement de ladite composition, pouvant être caractérisée par des observations empiriques, telle que l'observation visuelle de l'écoulement de la composition et/ou par la mesure d'une force maximale de compression mise en oeuvre à l'aide d'un texturomètre muni d'une sonde de mesure et relié à un logiciel de traitement des données.

**II - 2 - 1 -** Evaluation de la consistance de la composition par observation visuelle

a) - Principe de la méthode

La consistance peut être évaluée par une observation visuelle de l'écoulement de la composition évaluée qui est contenue dans un flacon suite à une rotation de 180° dudit flacon.

b) - Protocole expérimental

Une quantité de 50 grammes de la composition testée est introduite dans un flacon en verre transparent de 100 ml, qui est fermé à l'aide d'un bouchon à vis. L'expérimentateur dûment entrainé et expérimenté retourne à 180° le flacon contenant la composition testée pendant une durée de 3 secondes et note son observation. Il fait alors subir une nouvelle rotation de 180° au flacon contenant la composition testée.

c) - Expression des résultats

- Si pendant la durée de 3 secondes, pendant laquelle le flacon contenant est retourné de 180°, la composition testée s'écoule spontanément, la composition à usage est qualifiée de "liquide".
- Si pendant la durée de 3 secondes, pendant laquelle le flacon contenant est retourné de 180°, la composition testée ne s'écoule pas spontamént mais s'écoule à la suite de trois impulsions manuelles données sur le fond du flacon par l'expérimentateur, la composition cosmétqiue est qualifiée de "coulable".
- Si pendant la durée de 3 secondes, pendant laquelle le flacon contenant est retourné de 180°, la composition testée ne s'écoule pas spontanément et si elle ne s'écoule pas à la suite de trois impulsions manuelles données sur le fond du flacon par l'expérimentateur, la composition testée est qualifiée de "compacte".

**II - 2 - 2 -** Evaluation de la consistance d'une composition par la mesure d'une force maximale de compression

a) - Principe de la méthode

La consistance peut être évaluée par la mesure d'une force maximale de compression, mise en oeuvre à l'aide d'un texturomètre de modèle TEC95 commercialisé par la société ETIA, et muni d'une sonde hémisphérique de mesure et relié à un logiciel de traitement des données.

b) - Protocole expérimental

La composition testée est introduite dans un flacon de diamètre intérieur de 7,7 centimètres et de hauteur de 4 centimètres, qui après fermeture hermétique est conservé pendant 24 heures à une température de 20°C. Le texturomètre de modèle TEC95 est constitué d'une unité de translation sur laquelle est montée une sonde hémisphérique en inox, qui mesure en continu, par l'intermédiaire d'un capteur de force de 50 Newtons $\pm$ 0,025 Newtons, **la force subie par l'échantillon** par l'échantillon lors du mouvement de translation de la sonde. Le mouvement de translation de la sonde consiste en un simple mouvement de descente de la sonde dans l'échantillon. La vitesse de translation est réglée à 1 millimètre/seconde, le seuil de déclenchement pour la mesure **de la force** subie par l'échantillon est de 0,1 Newtons, et la profondeur d'enfoncement de la sonde dans l'échantillon est réglé à 10 millimètres. Après cette durée de stockage de 24 heures, le flacon contenant la composition à tester est ouvert et soumis aux conditions expérimentales indiquées ci-dessous pour mesurer la force maximale de compression. Lors de la mesure, l'évolution des forces est enregistrée par le logiciel ETIA relié au texturomètre qui permet d'enregistrer une courbe d'évolution des forces en fonction de la durée d'enfoncement et de calculer grace à cette courbe d'évolution la force maximale de compression exprimée en Newton.

c) - Expression des résultats

- Si la force maximale de compression, mesurée dans les conditions expérimentales telles que décrites ci-dessus, est supérieure ou égale à une valeur de 0,3 Newtons, la composition testée est considérée comme "compacte".
- Si la force maximale de compression, mesurée dans les conditions expérimentales telles que décrites ci-dessus, est supérieure ou égale à une valeur de 0,2 Newtons et strictement inférieure à une valeur de 0,3 Newtons, la composition testée est considérée comme "coulable".
- Si la force maximale de compression, mesurée dans les conditions expérimentales telles que décrites ci-dessus, est strictement inférieure à une valeur de 0,2 Newtons, la composition testée est considérée comme "liquide".

**II - 3 - Evaluation du critère de douceur procuré par une composition.**

Par critère de douceur procuré par une composition, on entend la sensation de douceur que procure la composition au consommateur pendant son application et son étalement sur la peau.

a) Principe de la méthode

Le critère de douceur procuré par une composition est évalué par la détermination d'un indice de douceur à la suite d'une opération d'application de ladite composition sur la peau par un panel de 11 personnes, dûment entrainées à cette évaluation, qui classent sur une échelle allant de 0 à 10 la sensation procurée par l'application de la composition testée sur la peau. A à une composition jugée comme « très douce » lors de son application, correspondra un indice de douceur de 10, et à une composition jugée comme « pas douce » ou « rêche » lors de son application sur la peau correspondra un indice de douceur de 0.

b) Protocole expérimental

La surface de la peau de l'expérimentateur sur laquelle doit être appliquée la composition à évaluer est préalablement nettoyée avec de l'éthanol puis séchée à l'aide d'un papier absorbant. 50 µl de composition à évaluer sont prélevés à l'aide d'une micropipette puis déposés sur la peau de l'intérieur du poignet de l'expérimentateur. L'expérimentateur effectue ensuite des rotations d'environ trois centimètres avec deux doigts (index et majeur) de son autre main avec une fréquence d'environ une rotation par seconde.

L'expérimentateur caractérise ensuite la sensation de douceur que lui a procuré l'application de la composition testée par l'attribution de l'indice de douceur compris 5 dans une échelle allant de 0 à 10.

c) Expression des résultats

Pour chaque composition testée, on relève les indices de douceur notés par chacun des 11 expérimentateurs et on calcule ensuite la moyenne arithmétique selon la formule suivante :

indice de douceur moyen = Somme des indices de douceur de chaque expérimentateur/11.

**II - 4 -** Résultats obtenus pour les émulsions (E1), (E'1), (E2), (E'2) et (E3)

Les résultats de l'évaluation des propriétés sensorielles de l'émulsion (E1) selon l'invention et des émulsions (E'1), (E2), (E'2) et (E3) selon l'état de la technique, à savoir l'effet « savonnant », la consistance et l'indice de douceur sont consignés dans le tableau 3 ci-dessous.

**Tableau 3**

|  | (E1) | (E'1) | (E'2) | (E2) | (E3) |
|---|---|---|---|---|---|
|  | **Effet « savonnant »** | | | | |
| Nombre de rotations moyen - | 87 | 56 | 70 | 9 | 16 |
|  | **Consistance** | | | | |
| Observation visuelle | Compacte | Compacte | Coulable | Coulable | liquide |

(suite)

| | Consistance | | | | |
|---|---|---|---|---|---|
| Force maximale de compression | 0,45 N | -<br>- | -<br>- | 0,5 N | 0.15 N |
| | Douceur | | | | |
| Indice de douceur | 9 | 4,4 | 5,5 | 9 | 6 |

L'émulsion (E1) selon l'invention et les émulsions (E'1) et (E2) selon l'état de la technique présentent une consistance « compacte » alors que l'émulsion (E'2) présente une consistance « coulable » et l'émulsion (E3) présente une consistance jugée « liquide ».

Les émulsions (E1), (E2) et (E3) présentent un indice de douceur supérieur à 5. alors que les émulsions (E'1) et (E'2) présentent un indice de douceur inférieur à 5.

Les émulsions (E1) et (E2) présentent un indice de douceur supérieur à 7, contrairement aux émulsions (E'1), (E'2) et (E3) qui présentent respectivement un indice de douceur de 4,4, de 5,5 et de 6,0.

Les émulsions (E2) et (E3) de l'état de la technique se caractérisent cependant par un effet savonnant important, car le nombre moyen de rotations nécessaire à l'apparition d'un film blanchâtre sur la peau est respectivement de 9 et de 16, alors que l'émulsion (E1) est classée « non savonnante » selon le protocole mis en oeuvre car le nombre moyen de rotations nécessaire à l'apparition d'un film blanchâtre sur la peau est de 87. L'émulsion (E'1) selon l'état de la technique est classée « peu savonnante » selon le protocole mis en oeuvre car le nombre moyen de rotations nécessaire à l'apparition d'un film blanchâtre est de 56, ne remplissant pas ainsi l'exigence recherchée pour cette propriété spécifique.

L'émulsion (E'2) qui ne se différencie de l'émulsion (E1) selon l'invention que par la nature de la phase grasse (l'émulsion (E1) comprend de l'huile d'amande douce et l'émulsion (E'2) comprend l'huile de jojoba), montre un effet « non savonnant » satisfaisant mais une consistance et un indice de douceur qui ne satisfont pas les exigences requises.

La composition selon l'invention (E1) présente donc des propriétés sensorielles améliorées par rapport aux émulsions (E'1), (E2), (E'2) et (E3) de l'état de la technique.

**III** - **Mise en évidence de l'amélioration des propriétés sensorielles d'une émulsion huile-dans-eau auto-bronzante.**

**III** - **1 - Préparation d'émulsions huile-dans-eau autobronzantes**

On prépare trois émulsions huile-dans-eau autobronzantes (E4), (E5), (E6) selon le même mode opératoire suivant :

Les émulsions (E4), (E5) et (E6) sont préparées en utilisant le mode opératoire suivant :

- Les composants de la phase grasse sont introduits successivement dans un réacteur et homogénéisés à une température de 80°C pendant 15 minutes à l'aide d'un agitateur muni d'un mobile de type « ancre » à une vitesse d'agitation de 80 tours/minute.
- Le système émulsionnant est alors ajouté sur la phase grasse à une température de 80°C, et le mélange ainsi obtenu est homogénéisé pendant 30 minutes par l'intermédiaire d'un agitateur mécanique muni d'un mobile de type ancre, à une vitesse de 80 tours/minute.
- La phase aqueuse comprenant l'eau, le glycérol, le propylène glycol et le polymère épaississant (KELTROL™ CG T) est préparée séparément par mélange de ses différents constituants dans un bécher à une température de 25°C sous agitation mécanique par l'intermédiaire d'un agitateur muni d'un mobile de type « ancre » à une vitesse de 80 tours/minutes. Une telle phase aqueuse est ensuite portée à une température de 80°C et maintenue à cette température sous agitation pendant 15 minutes.
- La phase aqueuse est ensuite ajoutée sur le mélange comprenant la phase grasse et l'agent émulsionnant à une température de 80°C.
- Le mélange résultant est ensuite cisaillé à l'aide d'un émulseur de type « rotor-stator », commercialisé par la société SILVERSON, pendant une durée de 4 minutes à une vitesse de 4000 tours/minute, à une température de 80°C.
- L'émulsion obtenue est ensuite agitée pendant 30 minutes à l'aide d'un agitateur muni d'un mobile de type « ancre » à une vitesse d'agitation de 100 tours/minute, puis mise à refroidir
- Lorsque la température atteint 40°C, la solution de dihydroxyacétone à 50% dans l'eau est ajoutée ainsi que l'agent conservateur GEOGARD™™ 221. Lorsque la température atteint 20°C, l'émulsion obtenue est maintenue 15 minutes sous agitation à l'aide d'un agitateur muni d'un mobile de type « ancre » à une vitesse d'agitation de 100 tours/minute.

Les compositions massiques de chacune des émulsions sont consignées dans le tableau 4ci-dessous.

**Tableau 4**

|  | (E4) | (E5) | (E6) |
|---|---|---|---|
| **Système émulsionnant** |  |  |  |
| - Composition (A1) | 2% | 0% | 0% |
| - MONTANOV™ 68[1] | 0% | 2% | 0% |
| - MONTANOV™ 202[9] | 0% | 0% | 2% |
| **Phase grasse** |  |  |  |
| Triglycéride C8-C10 | 15% | 15% | 15% |
| DERMOFEEL™™ Toco 70[7] | 0,05% | 0,05% | 0,05% |
| **Phase aqueuse** |  |  |  |
| GEOGARD™™ 221[4] | 0,6% | 0,6% | 0,6% |
| Glycérine | 3% | 3% | 3% |
| Propylène glycol | 2% | 2% | 2% |
| Eau | Qsp 100% | Qsp 100% | Qsp 100% |
| KELTROL™ CG T[6] | 0,2% | 0,2% | 0,2% |
| **Agent autobronzant** |  |  |  |
| Dihydroxyacétone en solution à 50% dans l'eau | 10% | 10% | 10% |
| (9) : Composition émulsionnante (arachidyl glucoside, alcool arachidylique + alcool béhènylique). | | | |

**III - 2 - Evaluation des propriétés des émulsions huile-dans-eau autobronzantes (E4), (E5) et (E6)**

Les émulsions (E4, (E5) et (E6) ainsi préparées sont ensuite conservées dans une enceinte climatique isolée et régulée à une température de 20°C pendant 7 jours. A l'issue de cette période de 7 jours, l'aspect de chaque émulsion préparée est observé et la viscosité de chaque émulsion est mesurée. Les émulsions sont ensuite replacées et conservées dans la même enceinte climatique isolée et régulée à une température de 20°C pendant une durée de 1 mois. Après une période d'un mois, chaque émulsion est sortie de l'enceinte climatique pour en observer l'aspect.

Les émulsions (E4), (E5) et (E6) sont caractérisées par :

- l'évaluation de leurs propriétés sensorielles selon les protocoles décrits dans les paragraphes II-1, II-2 et II-3 de la présente demande ;
- l'observation de leur aspect visuel après stockage pendant trois mois à une température de 20°C ;
- l'observation de leur aspect visuel après stockage pendant trois mois à une température de 45°C.

Les résultats obtenus sont indiqués dans le tableau 5 ci-dessous :

**Tableau 5**

|  | (E4) | (E5) | (E6) |
|---|---|---|---|
|  | **Effet « savonnant »** | | |
| Nombre de rotations moyen | 94 | 13 | 18 |
|  | Consistance | | |
| Observation visuelle | Compacte | Compacte | Coulable |
| Force maximale de compression | 0,3 N | 0,35 N | 0,2 N |
|  | **Douceur** | | |
| Indice de douceur | 9 | 9 | 8 |

Les émulsions autobronzantes (E5) et (E6) de l'état de la technique se caractérisent par un indice de douceur respectivement de 9 et de 8, et par une consistance évaluée respectivement comme « compacte » et « coulable».

Les émulsions autobronzantes (E5) et (E6) de l'état de la technique se caractérisent cependant par un effet savonnant important, car le nombre moyen de rotations nécessaire à l'apparition d'un film blanchâtre sur la peau est respectivement de 13 et de 18.

L'émulsion autobronzante (E4) selon l'invention est classée « non savonnante » selon le protocole mis en oeuvre car le nombre moyen de rotations nécessaire à l'apparition d'un film blanchâtre sur la peau est de 94.

Par ailleurs, l'émulsion autobronzante (E4) selon l'invention se caractérise par un indice de douceur de 9, et par une consistance jugée « compacte ».

Par conséquent, l'émulsion autobronzante (E4) selon l'invention présente des propriétés sensorielles améliorées par rapport à celles procurées par les émulsions (E5) et (E6) de l'état de la technique.

### IV - Formulations

Dans les formules suivantes, les pourcentages sont exprimés en pourcentage massique pour 100% de la masse de la formulation.

### Exemple IV-1 Formulation fluide de jour

Formule

| | | |
|---|---|---|
| A | Composition (A1) | 3,00% |
| | MONTANOV™ 14 | 1,50% |
| | DUB™ BB | 2,00% |
| | Butyrospermum Parkj (Bio) | 1,50% |
| | DUB™MUG | 1,50% |
| | Squalane | 3,00% |
| | Caprylic/capric triglycéride | 6,00% |
| | DUB™ISIP | 3,00% |
| | DERMOFEEL™ Toco 70 | 0,10% |
| B | Eau | Q.S. 100,00% |
| | Aqueous Hordeum Vulgare extract | 3,50% |
| | Xantham Gum | 0,60% |
| C | GEOGARD™ 221 | 0,60% |
| | AQUAXYL™ | 3,00% |
| | DERMOSOFT™ 700 | 0,50% |
| | Soude | Q.S. pH = 5,5 |

Mode opératoire : la phase B est ajoutée sur la phase A à une température de 80°C, sous agitation avec une turbine de type rotor/stator, puis refroidie sous agitation modérée avec un agitateur mécanique muni d'un mobile d'agitation de type ancre à une vitesse de 80 toures/minute. La phase C est ensuite ajoutée dans les mêmes conditions d'agitation à une température de 40°C.

### Exemple IV-2 Lait démaquillant dermopurifiant

Formule

| | | |
|---|---|---|
| A | Eau | Q.S. 100,00% |
| | Amigum (Sclerotium gum) | 0,15% |
| B | Eau | Q.S. 30,00% |

(suite)

| | | | |
|---|---|---|---|
| | | LIPACIDE™C8G | 1,00% |
| | | Soude à 48% | Q.S. pH = 5,5 |
| | | PROTEOL™OAT | 2,00% |
| | | Glycérol | 2,00% |
| | C | Composition (A1) | 4,00% |
| | | Caprylic/capric triglycéride | 4,00% |
| | | Squalane | 4,00% |
| | | Corylus Nucifera (Hazel) seed oil | 2,00% |
| | | Prunus Amygdalus Dulcis (Sweet almond) oil | 2,00% |
| | | Cire d'abeille | 0,30% |
| | D | Aqueous Hordeum Vulgare extract | 10,00% |
| | | GEOGARD™ 221 | 0,60% |
| | | DERMOFEEL™ Toco 70 | 0,10% |

Mode opératoire :les phases A et B sont préalablement préparées séparement, puis la phase A est ajoutée sur la phase B à une température de 80°C sous agitation mécanique avec un agitateur muni d'un moobile de type ancre à une vitesse de 80 tours/minutes. La phase C est ensuite ajoutée au mélange des phase A+B à une temérature de 80°C sous agitation avec une turbine rotor/stator puis refroidie sous agitation modérée. La phase D est ajoutée ensuite à 40°C.

**Exemple IV-3 Lait intense fermeté pour le corps**

Formule

| | | | |
|---|---|---|---|
| A | Eau | Q.S. 100,00% | |
| | Soude à 48% | Q.S. pH = 5,5 | |
| | AVICEL™PC 611 | 0,40% | |
| | Glycérol | 2,00% | |
| B | Composition (A1) | 2,00% | |
| | DUB™ISIP | 4,00% | |
| | Caprylic/capric triglycéride | 7,00% | |
| | Carthamus Tinctorius (Safflower) oil | 1,00% | |
| | SEPILIFT™DPHP | 1,00% | |
| C | Aqueous Hordeum Vulgare extract | 10,00% | |
| D | GEOGARD™ 221 | 0,60% | |
| | DERMOFEEL™ Toco 70 | 0,10% | |
| | Fragrance | 0,10% | |

Mode opératoire : la phase A est ajoutée sur la phase B à 80°C sous agitation avec une turbine rotor/stator. Le mélange obtenu est ensuite refroidi jusqu'à une température de 40°C sous agitation modérée à l'aide d'un agitateur mécanique muni d'un mobile de type ancre à une vitesse de 80 tours/minute. La phase C est ensuite ajoutée à 40°C, puis la phase D est alors ajouté à 40°C.

Le MONTANOV™ 14 est un mélange d'alcool myristique et de myristyl polyglucosides commercialisé par la société SEPPIC comme agent émulsionnant.

Le DUB™ BB est le béhénate de béhényle commercialisé par la société STEARINERIE DUBOIS.

Le DUB™MUG est l'undécylénate de glycérol commercialisé par la société STEARINERIE DUBOIS.

Le DUB™ISIP est l'isopropyl isostéarate commercialisé par la société STEARINERIE DUBOIS.

Le DERMOFEEL™ Toco 70 est un mélange de tocophérols et d'huile de graines d'Hélianthus Annus utilisé comme antioxydant et commercialisé par la société Dr Straetmans.

LeGEOGARD™ 221 est un mélange d'acide dehydroacétique et d'alcool benzylique utilisé comme agent conservateur et commercialisé par la société LONZA.

Le LIPACIDE™C8G est le capryloyl glycine commercialisé par la société SEPPIC comme agent actif dermopurifiant.

Le PROTEOL™OAT est un mélange de N-lauryl aminoacides obtenus par hydrolyse totale de protéine d'avoine tel que décrit dans WO 94/26694 et commercialisé par la société SEPPIC.

AVICEL™PC 611 est de la cellulose microcristalline commercialisée par la société FMC.

Le SEPILIFT™DPHP est le DiPalmitoylHydroxyProline commercialisé par la société SEPPIC comme ingrédient actif anti-rides.

**Revendications**

1. Composition se présentant sous la forme d'une émulsion huile-dans-eau comprenant pour 100% de sa masse :

   a) - De 0,1% à 10% massique d'un système émulsionnant (A) consistant pour 100% de sa masse en :

   (i) - De 5% massique à 95% massique du mélange des produits de la réaction d'un sucre réducteur et du 1,12-octadécanediol constitué essentiellement d'hydroxyoctadécyl polyglycosides de formule (I) :

   $$HO\text{-}R\text{-}O\text{-}(G)_n\text{-}H \qquad (I)$$

   et de polyglycosyloctadécylpolyglycosides de formule (II) :

   $$H\text{-}(G)_m\text{-}O\text{-}R\text{-}O\text{-}(G)_p\text{-}H \qquad (II),$$

   formules (I) et (II) dans lequelles G représente le reste dudit sucre réducteur et R représente le radical divalent octadécane-diyl-1,12 et dans lesquelles n, m et p, identiques ou différents, représentent chacun, indépendamment les uns des autres, un nombre décimal compris entre 1 et 5 ;
   (ii) - De 95% massique à 5% massique de 1,12-octadécanediol ;

   b) - De 0,01% à 5% massique d'au moins un agent épaississant et/ou gélifiant choisi parmi les agents épaississants et/ou gélifiants d'origine naturelle ;
   c) - De 10% à 50% massique d'une phase grasse (P2) constituée d'une ou plusieurs huiles végétales à base de monoglycérides et/ou de diglycérides et/ou de triglycérides ; et
   d) - De 89,89% massique à 35% massique d'une phase aqueuse (P1) cosmétiquement acceptable.

2. Composition telle que définie à la revendication 1, **caractérisée en ce qu'**elle comprend en outre pour 100% de sa masse, de 0,5% massique à 10% massique de dihydroxyacétone.

3. Utilisation cosmétique de la composition telle que définie à la revendication 1 pour le nettoyage, pour la protection et/ou pour le soin de la peau, des cheveux ou du cuir chevelu.

4. Utilisation cosmétique de la composition telle que définie à la revendication 2, pour bronzer et/ou brunir artificiellement la peau.

5. Procédé de traitement cosmétique pour bronzer et/ou brunir artificiellement la peau, **caractérisé en ce que** l'on applique sur celle-ci la composition telle que définie à la revendication 2.

6. Procédé de préparation de la composition telle que définie à l'une des revendications 1 ou 2, comprenant :

   au moins une étape a) de mélange de la phase grasse (P2) avec le système émulsionnant (A) ;
   au moins une étape b) d'émulsification du mélange de la phase grasse (P2) et du système émulsionnant (A) obtenu à l'issue de l'étape a) avec la phase aqueuse (P1).

**Patentansprüche**

1. Zusammensetzung, die in Form einer Öl-in-Wasser-Emulsion vorliegt und die pro 100 % ihrer Masse Folgendes enthält:

a) 0,1 Masse-% bis 10 Masse-% eines Emulgatorsystems (A), pro 100 % seiner Masse bestehend aus:

(i) 5 Masse-% bis 95 Masse-% des Gemischs aus den Produkten der Reaktion eines reduzierenden Zuckers und aus 1,12-Octadecandiol, das im Wesentlichen aus Hydroxyoctadecyl-Polyglycosiden der Formel (I)

$$HO\text{-}R\text{-}O\text{-}(G)_n\text{-}H \qquad (I)$$

und aus Polyglycosyloctadecyl-Polyglycosiden der Formel (II) $H\text{-}(G)_m\text{-}O\text{-}R\text{-}O\text{-}(G)_p\text{-}H$ (II) besteht, wobei in den Formeln (I) und (II) G den Rest des reduzierenden Zuckers darstellt und R das zweiwertige 1,12-octadecandiyl-Radikal darstellt und in denen n, m und p, die identisch oder verschieden sind, jeweils, unabhängig voneinander, eine Dezimalzahl zwischen 1 und 5 darstellen;
(ii) 95 Masse-% bis 5 Masse-% 1,12-octadecandiol;

b) 0,01 Masse-% bis 5 Masse-% mindestens eines Verdickungsmittels und/oder eines Geliermittels, das aus den Verdickungsmitteln und/oder Geliermitteln natürlichen Ursprungs gewählt ist;
c) 10 Masse-% bis 50 Masse-% einer Fettphase (P2), die aus einem oder mehreren pflanzlichen Ölen auf der Basis von Monogleriden und/oder Diglyceriden und/oder Triglyceriden besteht; und
d) 89,89 Masse-% bis 35 Masse-% einer kosmetisch unbedenklichen wässrigen Phase (P1).

**2.** Zusammensetzung nach Anspruch 1, **dadurch gekennzeichnet, dass** sie ferner pro 100 % ihrer Masse 0,5 Masse-% bis 10 Masse-% Dihydroxyaceton enthält.

**3.** Kosmetische Verwendung der Zusammensetzung nach Anspruch 1 zur Reinigung, zum Schutz und/oder zur Pflege der Haut, der Haare oder der Kopfhaut.

**4.** Kosmetische Verwendung der Zusammensetzung nach Anspruch 2 zum Bräunen und/oder künstlichen Braunfärben der Haut.

**5.** Verfahren zur kosmetischen Behandlung zur Bräunung und/oder künstlichen Braunfärbung der Haut, **dadurch gekennzeichnet, dass** auf diese die Zusammensetzung nach Anspruch 2 aufgetragen wird.

**6.** Verfahren zur Herstellung der Zusammensetzung nach einem der Ansprüche 1 bis 2, umfassend
mindestens einen Schritt a) des Mischens der Fettphase (P2) mit dem Emulgatorsystem (A);
mindestens einen Schritt b) des Emulgierens des am Ende von Schritt a) erhaltenen Gemischs aus der Fettphase (P2) und aus dem Emulgatorsystem (A) mit der wässrigen Phase (P1).

**Claims**

**1.** Composition in the form of an oil-in-water emulsion, comprising, per 100 % of its weight:

a) - from 0.1 wt.% to 10 wt.% of an emulsifying system (A) consisting per 100 % of its weight of:

(i) - from 5 wt.% to 95 wt.% of the mixture of the reaction products of a reducing sugar and of 1,12-octadecanediol consisting substantially of hydroxyoctadecyl polyglycosides of formula (I):

$$HO\text{-}R\text{-}O\text{-}(G)_n\text{-}H \qquad (I)$$

and of polyglycosyloctadecyl polyglycosides of formula (II):

$$H\text{-}(G)_m\text{-}O\text{-}R\text{-}O\text{-}(G)_p\text{-}H \qquad (II),$$

wherein G represents the residue of said reducing sugar and R represents the 1,12-octadecane-diyl divalent radical and wherein n, m and p, which may be identical or different, each represent, independently of one another, a decimal number between 1 and 5;
(ii) - from 95 wt.% to 5 wt.% of 1,12-octadecanediol;

b) - from 0.01 wt.% to 5 wt.% of at least one thickener and/or gelling agent selected from thickeners and/or gelling agents of natural origin;

c) - from 10 wt.% to 50 wt.% of a fatty phase (P2) consisting of one or more vegetable oils based on monoglycerides and/or diglycerides and/or triglycerides; and

d) - from 89.89 wt.% to 35 wt.% of a cosmetically acceptable aqueous phase (P1).

2. Composition according to claim 1, **characterised in that** it further comprises from 0.5 wt.% to 10 wt.% of dihydroxyacetone per 100 % of its weight.

3. Cosmetic use of the composition according to claim 1 for cleansing, protecting and/or for caring for the skin, hair or scalp.

4. Cosmetic use of the composition according to claim 2 for artificially bronzing and/or tanning the skin.

5. Cosmetic treatment method for artificially bronzing and/or tanning the skin, **characterised in that** the composition according to claim 2 is applied thereto.

6. Method for preparing the composition according to either claim 1 or claim 2, comprising:

at least one step a) of mixing the fatty phase (P2) with the emulsifying system (A);
at least one step b) of emulsifying the mixture, obtained in step a), of the fatty phase (P2) and the emulsifying system (A) with the aqueous phase (P1).

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 9206778 A
- WO 9513863 A
- WO 9637285 A
- WO 0056438 A
- FR 2830464
- FR 2803537
- US 6348 A
- US 202 B1
- WO 9401073 A
- EP 0958856 A1
- WO 9600719 A
- WO 9426694 A

**Littérature non-brevet citée dans la description**

- Jojoba. **D.J. UNDERSTANDER ; E.A. OELKE ; A.R. KAMINSKI ; J.D. DOLL ; S.M. COMBS ; C.V. HANSON,.** Alternative Field Crops Manual. 1990
- **DANIEL VOET ; JUDITH G. VOET.** Biochemistry. John Wyley & Sons, 1990, 250